(19) Europäisches Patentamt
European Patent Office
Office européen des brevets

(11) EP 3 777 826 A1

(12) EUROPEAN PATENT APPLICATION
published in accordance with Art. 153(4) EPC

(43) Date of publication:
17.02.2021 Bulletin 2021/07

(51) Int Cl.:
A61K 8/81 (2006.01)        A61Q 19/10 (2006.01)

(21) Application number: 19777907.7

(22) Date of filing: 27.03.2019

(86) International application number:
PCT/JP2019/013115

(87) International publication number:
WO 2019/189325 (03.10.2019 Gazette 2019/40)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 27.03.2018   JP 2018059401

(71) Applicant: Sumitomo Seika Chemicals Co., Ltd.
Kako-gun, Hyogo 675-0145 (JP)

(72) Inventors:
• YAMAMOTO, Tomoe
  Kako-gun Hyogo 675-0145 (JP)
• KANKI, Toshihiko
  Kako-gun Hyogo 675-0145 (JP)

(74) Representative: Mewburn Ellis LLP
Aurora Building
Counterslip
Bristol BS1 6BX (GB)

(54) **WATER-ABSORBING EXFOLIATOR, METHOD FOR PRODUCING SAME, AND COSMETIC**

(57) Provided is an exfoliator which absorbs water and exhibits suitable hardness, provides little skin stimulation, and has a favorable skin massaging effect. The exfoliator exhibits a pure-water absorption factor of at least 30 times, and exhibits a compression-breaking stress of 0.14-1.40N upon absorbing water and swelling to 30 times the initial mass thereof.

EP 3 777 826 A1

**Description**

TECHNICAL FIELD

The present invention relates to a water-absorbing exfoliator, a method for producing the water-absorbing exfoliator, and a cosmetic.

BACKGROUND ART

Conventionally, exfoliators are sometimes blended with cosmetics such as skin cleaning agents from the viewpoint of, for example, improving the cleaning ability. In improving the cleaning ability by an exfoliator, the following two actions are mainly involved. The first action of the exfoliator is to scrape off dirt by hitting the dirt, and the second action of the exfoliator is to remove dirt by thickening and adhering to the dirt.

Examples of the cosmetic in which an exfoliator is widely used include face washes. In face washes, exfoliators are mainly used for removing sebum and old keratin in pores.

Exfoliators are also used in cosmetics other than cleaning agents for the purpose of imparting a massage effect by stimulation caused by an exfoliator hitting the skin. Examples of such a cosmetic include body creams and massage creams.

As conventional exfoliators to be blended with cosmetics, for example, particles of activated carbon, pulverized products of talc (clay minerals such as hydrotalcite), particles of a synthetic resin such as polyethylene, and spherical porous silica are known (see, for example, Patent Documents 1 and 2). Furthermore, as the exfoliators, granular exfoliators in which activated carbon powder or talc powder is granulated with a water-soluble resin and the shape is deformed by stress, or gel-like exfoliators in which a water-soluble polymer is granulated or crosslinked are also known.

PRIOR ART DOCUMENTS

PATENT DOCUMENTS

[0006]

　　Patent Document 1: Japanese Patent Laid-open Publication No. 06-179892
　　Patent Document 2: Japanese Patent Laid-open Publication No. 2011-225548

SUMMARY OF THE INVENTION

PROBLEMS TO BE SOLVED BY THE INVENTION

Although the exfoliators such as particles of activated carbon, pulverized products of talc, particles of a synthetic resin such as polyethylene, and spherical porous silica can exert a high cleaning effect when blended with a cleaning agent, there is a problem that the exfoliators have strong skin irritation because of the hard particles. Furthermore, the above-described granular exfoliators are deformed immediately when an external force is applied, therefore, although the skin irritation such as pain is small, a skin massage effect is difficult to obtain. The above-described gel-like exfoliators are soft, therefore, although the skin irritation such as pain is small, a skin massage effect is difficult to obtain.

A main object of the present invention is to provide a water-absorbing exfoliator that absorbs water, exhibits suitable hardness, provides little skin irritation, and has a favorable skin massage effect.

MEANS FOR SOLVING THE PROBLEM

The present inventors intensively studied to solve the above-described problems. As a result, it has been found that a water-absorbing resin particle, that has a pure-water absorption factor of 30 times or more and a compression-breaking stress of 0.14 to 1.40 N in a state that the water-absorbing resin particle is swollen to have a mass 30 times of the initial mass by absorbing water, absorbs water, exhibits suitable hardness, provides little skin irritation, and has a favorable skin massage effect. The present invention has been completed based on such findings.

That is, the present invention provides an invention having the following configuration.

　　Item 1. A water-absorbing exfoliator characterized by a pure-water absorption factor of 30 times or more, and a compression-breaking stress of 0.14 to 1.40 N in a state that the water-absorbing exfoliator is swollen to have a mass 30 times of an initial mass of the water-absorbing exfoliator by absorbing water.

Item 2. The water-absorbing exfoliator according to Item 1, having a structure in which a second polymer is infiltrated into a first polymer particle.

Item 3. The water-absorbing exfoliator according to Item 1 or 2, wherein

the first polymer particle includes a polymer of a first monomer component including at least one of a monomer A or a salt of the monomer A,

the second polymer includes a polymer of a second monomer component including at least one of a monomer B or a salt of the monomer B, and

the monomer A has an acid dissociation index smaller than an acid dissociation index of the monomer B.

Item 4. The water-absorbing exfoliator according to Item 3, wherein a difference between the acid dissociation index of the monomer B and the acid dissociation index of the monomer A (ΔpKa) is 1.5 or more.

Item 5. The water-absorbing exfoliator according to Item 4, wherein

the monomer A is an unsaturated sulfonic acid-based monomer, and

the monomer B is a water-soluble ethylenically unsaturated monomer.

Item 6. The water-absorbing exfoliator according to any one of Items 1 to 5, having a granular shape, a substantially spherical shape, or a shape in which substantially spherical particles are aggregated.

Item 7. A method for producing a water-absorbing exfoliator, the method including the steps of:

preparing a first polymer particle;

infiltrating a second monomer component that is to form a second polymer and includes at least one of a monomer B or a salt of the monomer B into the first polymer particle; and

polymerizing the second monomer component infiltrated into the first polymer particle to form a structure in which the second polymer is infiltrated into the first polymer particle.

Item 8. A cosmetic including the water-absorbing exfoliator according to any one of Items 1 to 6.

ADVANTAGES OF THE INVENTION

According to the present invention, it is possible to provide a water-absorbing exfoliator that absorbs water, exhibits suitable hardness, provides little skin irritation, and has a favorable skin massage effect. Furthermore, according to the present invention, it is also possible to provide a method for producing the water-absorbing exfoliator, and a cosmetic including the water-absorbing exfoliator.

EMBODIMENTS OF THE INVENTION

The water-absorbing exfoliator according to the present invention is characterized by a pure-water absorption factor of 30 times or more, and a compression-breaking stress of 0.14 to 1.40 N in a state that the water-absorbing exfoliator is swollen to have a mass 30 times of an initial mass of the water-absorbing exfoliator by absorbing water. Because the water-absorbing exfoliator according to the present invention has such specific physical properties, the water-absorbing exfoliator can exhibit suitable hardness, provide little skin irritation, and exert a suitable skin massage effect when used in a cosmetic or the like.

The water-absorbing exfoliator according to the present invention can be suitably produced by, for example, a production method including the following steps. That is, the water-absorbing exfoliator according to the present invention is suitably produced by a production method including the steps of: preparing a first polymer particle; infiltrating a second monomer component that is to form a second polymer and includes at least one of a monomer B or a salt of the monomer B into the first polymer particle; and polymerizing the second monomer component infiltrated into the first polymer particle to form a structure in which the second polymer is infiltrated into the first polymer particle.

Hereinafter, the water-absorbing exfoliator according to the present invention, a method for producing the water-absorbing exfoliator, and a cosmetic including the water-absorbing exfoliator will be described in detail.

In the numerical range described stepwise in the present description, the upper limit or the lower limit in the numerical range of a certain stage can be optionally combined with the upper limit or the lower limit in the numerical range of another stage. In the numerical range described in the present description, the upper limit or the lower limit in the numerical range may be replaced with a value shown in Examples or a value that can be uniquely derived from Examples. In the present description, the numerical values connected by "to" means a numerical range including the numerical values before and after "to" as a lower limit and an upper limit.

(Water-Absorbing Exfoliator)

The water-absorbing exfoliator according to the present invention is a particulate water-absorbing resin. The water-absorbing exfoliator (particulate water-absorbing resin) preferably has a structure in which the second polymer is infiltrated into the first polymer particle. The term "structure in which the second polymer is infiltrated into the first polymer particle" means that the second polymer is present from the surface to the inner side of the first polymer particle, and refers to a structure formed by, for example, the method for producing the water-absorbing exfoliator described below.

In the water-absorbing exfoliator, the second polymer may be present only on the surface of the first polymer particle and in the vicinity of the surface, or may reach from the surface to the central portion. The second polymer covers at least a part of the surface of the first polymer particle, and may cover the entire surface or a part of the surface. The second polymer may connect the plurality of first polymer particles.

The water-absorbing exfoliator according to the present invention having the above-described physical properties can be produced by, for example, infiltrating the second monomer component that is to form the second polymer and includes at least one of the monomer B or a salt of the monomer B into the first polymer particle, and polymerizing the second monomer component infiltrated into the first polymer particle. The details of this method will be described below.

The lower limit of the pure-water absorption factor of the water-absorbing exfoliator is 30 times, preferably 70 times, and more preferably 100 times from the viewpoint that the water-absorbing exfoliator exhibits suitable hardness by absorbing water, provides little skin irritation, and has a favorable skin massage effect. The upper limit of the pure-water absorption factor is, for example, 400 times, preferably 300 times, and more preferably 200 times.

The pure-water absorption factor of the water-absorbing exfoliator is a value measured by the method described in Examples.

The compression-breaking stress of the water-absorbing exfoliator is 0.14 to 1.40 N from the viewpoint that the water-absorbing exfoliator exhibits suitable hardness by absorbing water, provides little skin irritation, and has a favorable skin massage effect. The lower limit of the compression-breaking stress is 0.14 N, preferably 0.17 N, and more preferably 0.2 N. The upper limit of the compression-breaking stress is preferably 1.13 N, more preferably 0.85 N, and still more preferably 0.60 N.

The compression-breaking stress of the water-absorbing exfoliator is a value measured in a state that the water-absorbing exfoliator is swollen to have a mass 30 times of the initial mass by absorbing pure water.

The compression-breaking stress of the water-absorbing exfoliator is measured by the method described in Examples.

The lower limit of the median particle diameter of the water-absorbing exfoliator is preferably 10 $\mu$m, more preferably 100 $\mu$m, still more preferably 130 $\mu$m, and particularly preferably 150 $\mu$m from the viewpoint that the water-absorbing exfoliator exhibits suitable hardness by absorbing water, provides little skin irritation, and has a favorable skin massage effect. The upper limit of the median particle diameter of the water-absorbing exfoliator is preferably 500 $\mu$m, more preferably 350 $\mu$m, still more preferably 250 $\mu$m, and particularly preferably 200 $\mu$m.

The median particle diameter of the water-absorbing exfoliator is a value measured using a JIS standard sieve, and specifically a value measured by the method described in Examples.

The shape of the water-absorbing exfoliator is, for example, a granular shape, a substantially spherical shape, a shape in which substantially spherical particles are aggregated, an irregularly crushed shape, a shape in which irregularly crushed particles are aggregated, a plate shape, or the like. In the case that the water-absorbing exfoliator is produced using a reverse phase suspension polymerization method or a spray droplet polymerization method, a water-absorbing exfoliator having a granular shape, a spherical shape, a substantially spherical particle shape such as an elliptic spherical shape, or a shape in which substantially spherical particles are aggregated is obtained. In the case that the water-absorbing exfoliator is produced using an aqueous solution polymerization method, a water-absorbing exfoliator having an irregularly crushed shape or a shape in which irregularly crushed particles are aggregated is obtained. The shape of the water-absorbing exfoliator is preferably a granular shape, a substantially spherical shape, or a shape in which substantially spherical particles are aggregated from the viewpoint that the water-absorbing exfoliator exhibits suitable hardness by absorbing water, provides little skin irritation, and has a favorable skin massage effect.

From the viewpoint that the water-absorbing exfoliator exhibits suitable hardness by absorbing water, provides little skin irritation, and has a favorable skin massage effect, in the water-absorbing exfoliator, it is preferable that the first polymer particle be a polymer of a first monomer component including at least one of a monomer A or a salt of the monomer A, the second polymer be a polymer of the second monomer component including at least one of the monomer B or a salt of the monomer B, and the monomer A have an acid dissociation index (pKa) smaller than the acid dissociation index of the monomer B. From the same viewpoint, the difference between the acid dissociation index of the monomer B and the acid dissociation index of the monomer A ($\Delta$pKa = acid dissociation index of monomer B - acid dissociation index of monomer A) is preferably 1.5 or more, more preferably 2.0 or more, and still more preferably 2.5 or more. Furthermore, $\Delta$pKa is, for example, 4.0 or less, preferably 3.5 or less, and more preferably 3.0 or less.

It can be considered that if the difference between the acid dissociation index of the monomer B and the acid dissociation index of the monomer A ($\Delta$pKa) is within the above-described range, the osmotic pressure of the first polymer particle is high, and the monomer B and/or its salt included in the second monomer component is difficult to ionize, so that the second monomer component is well infiltrated into the first polymer particle.

The acid dissociation index of the monomer A is preferably 0.5 to 2.5, more preferably 1.0 to 2.0, and still more preferably 1.0 to 1.5. The acid dissociation index of the monomer B is preferably 2.0 to 6.0, more preferably 3.5 to 5.0, and still more preferably 4.0 to 4.5.

The acid dissociation index (pKa) of the monomer A and that of the monomer B are values measured by the method described in Examples.

Preferable examples of the monomer A include unsaturated sulfonic acid-based monomers. Preferable examples of the monomer B include water-soluble ethylenically unsaturated monomers. Specific examples of the unsaturated sulfonic acid-based monomer, the water-soluble ethylenically unsaturated monomer, and the salts thereof will be shown in Method for Producing Water-Absorbing Exfoliator described below.

(Method for Producing Water-Absorbing Exfoliator)

In the method for producing the water-absorbing exfoliator, first, a first polymer particle is prepared. Next, a second monomer component that is to form a second polymer and includes at least one of a monomer B or a salt of the monomer B is infiltrated into the first polymer particle. In addition, the second monomer component infiltrated into the first polymer particle is polymerized to obtain a water-absorbing exfoliator having a structure in which the second polymer is infiltrated into the first polymer particle. Hereinafter, these steps will be described in detail.

The first polymer particle is prepared by polymerizing a first monomer component including at least one of a monomer A or a salt of the monomer A. In the case that the first monomer component includes only the monomer A and/or its salt, the first polymer particle has a structure in which only the monomer A and/or its salt is polymerized. In the case that the first monomer component includes a monomer, other than the monomer A and its salt, (hereinafter referred to as monomer X), the first polymer particle has a structure in which the monomer A and/or its salt, and, in addition, the monomer X are copolymerized. The monomer A and its salt are not particularly limited as long as after the first polymer particle is formed, the second monomer component that is to form the second polymer can be infiltrated into the first polymer particle and polymerized.

The first polymer particle is preferably a polymer in which the monomer A having the above-described acid dissociation index is used, more preferably a polymer in which an unsaturated sulfonic acid-based monomer is used as the monomer A, and more preferably a polymer in which an unsaturated sulfonic acid monomer having the above-described acid dissociation index is used as the monomer A.

The first polymer particle can be suitably produced by, for example, subjecting the first monomer component to reverse phase suspension polymerization in a hydrocarbon dispersion medium in the presence of an internal cross-linking agent and a radical polymerization initiator.

<Step of Polymerizing First Monomer Component>

[First Monomer Component]

The first monomer component that is to form the first polymer particle may include only the monomer A, may include only a salt of the monomer A, may include only the monomer A and its salt, or may include the monomer X in addition to the monomer A and/or its salt.

As the monomer A, a monomer satisfying the above-described acid dissociation index is preferable, and an unsaturated monomer of a strong electrolyte, such as unsaturated sulfonic acid-based monomer is preferable. Specific examples of the unsaturated sulfonic acid-based monomer include vinyl sulfonic acid, allyl sulfonic acid, methallyl sulfonic acid, styrene sulfonic acid, 2-(meth)acrylamido-2-methylpropane sulfonic acid, 3-allyloxy-2-hydroxypropane sulfonic acid, sulfoethyl (meth)acrylate, sulfopropyl (meth)acrylate, 2-hydroxysulfopropyl (meth)acrylate, sulfoethyl maleimide, and 3-sulfopropyl methacrylate, and 2-(meth)acrylamido-2-methylpropane sulfonic acid is preferably used. In the present description, "acry" and "methacry" are collectively referred to as "(meth)acry". Only one of the monomers A may be used, or two or more of the monomers A may be used in combination.

In the case that two or more monomers A are used in combination, it is preferable that one or some monomers A have an acid dissociation index in the above-described range, and it is more preferable that all monomers A have an acid dissociation index in the above-described range.

In the case that two or more monomers A are used in combination, the reference monomer A for determining the difference in acid dissociation index from the monomer B (ΔpKa) is the monomer A having the highest acid dissociation index of the two or more monomers A.

As for the monomer X, only one monomer X may be used, or two or more monomers X may be used in combination. For example, in the case that an unsaturated sulfonic acid-based monomer such as 2-(meth)acrylamido-2-methylpropanesulfonic acid is used as the monomer A, the monomer B described below (for example, (meth)acrylic acid and/or (meth)acrylamide as a water-soluble ethylenically unsaturated monomer) may be copolymerized as the monomer X. In the first monomer component, the proportion of the monomer A and/or its salt (in the case that the monomer A and its salt are included, the proportion of the total amount of the monomer A and its salt) is preferably 70 mol% or more, more preferably 80 mol% or more, and still more preferably 90 mol% or more. The upper limit of the proportion of the monomer A and/or its salt is 100 mol%. If the proportion of the monomer A and/or its salt is 100 mol%, the first monomer component includes no monomer X.

In the case that the monomer A, like 2-(meth)acrylamido-2-methylpropanesulfonic acid, has an acid group, the salt of the monomer A can be prepared by neutralizing the acid group with an alkaline neutralizing agent. Examples of the alkaline neutralizing agent include alkali metal salts such as sodium hydroxide, sodium carbonate, sodium hydrogen carbonate, potassium hydroxide, and potassium carbonate; and ammonia. The alkaline neutralizing agent may be used in the form of an aqueous solution in order to simplify the neutralizing operation. The above-described alkaline neutralizing agents may be used singly or in combination of two or more kinds thereof.

The degree of neutralization of the monomer A with the alkaline neutralizing agent is preferably 10 to 100 mol%, more preferably 30 to 100 mol%, still more preferably 40 to 100 mol%, and still even more preferably 50 to 100 mol% as the degree of neutralization based on all the acid groups of the monomer A.

The first monomer component is preferably dispersed in the form of an aqueous solution in a hydrocarbon dispersion medium and subjected to reverse phase suspension polymerization. The first monomer component in the form of an aqueous solution can have dispersion efficiency enhanced in the hydrocarbon dispersion medium. The concentration of the first monomer component in the aqueous solution is preferably in the range of 20% by mass to the saturated concentration. The concentration of the first monomer component is more preferably 55% by mass or less, still more preferably 50% by mass or less, and still even more preferably 45% by mass or less. Furthermore, the concentration of the first monomer component is more preferably 25% by mass or more, still more preferably 28% by mass or more, and still even more preferably 30% by mass or more.

[Hydrocarbon Dispersion Medium]

Examples of the hydrocarbon dispersion medium include aliphatic hydrocarbons having 6 to 8 carbon atoms such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. Among these hydrocarbon dispersion media, n-hexane, n-heptane, and cyclohexane are particularly preferably used because they are industrially easily available, stable in quality, and inexpensive. These hydrocarbon dispersion media may be used singly or in the form of a mixture of two or more kinds thereof. Examples of the mixture of the hydrocarbon dispersion media include commercial products such as EXXSOL Heptane (manufactured by Exxon Mobil Corporation: containing 75 to 85% by mass of heptane and its isomeric hydrocarbon).

The amount of the hydrocarbon dispersion medium used is preferably 100 to 1,500 parts by mass, and more preferably 200 to 1,400 parts by mass based on 100 parts by mass of the first monomer component used for the first stage polymerization. As will be described below, the reverse phase suspension polymerization is performed in one stage (single stage) or in multiple stages of two or more stages, and the term "first stage polymerization" described above means the polymerization reaction in the first stage of single stage polymerization or multistage polymerization (the same applies below).

[Dispersion Stabilizer]

In the reverse phase suspension polymerization, a dispersion stabilizer may be used in order to improve the dispersion stability of the first monomer component in the hydrocarbon dispersion medium.

(Surfactant)

A surfactant can be used as the dispersion stabilizer. As the surfactant, for example, sucrose fatty acid ester, polyglycerin fatty acid ester, sorbitan fatty acid ester, polyoxyethylene sorbitan fatty acid ester, polyoxyethylene glycerin fatty acid ester, sorbitol fatty acid ester, polyoxyethylene sorbitol fatty acid ester, polyoxyethylene alkyl ether, polyoxyethylene alkyl phenyl ether, polyoxyethylene castor oil, polyoxyethylene hydrogenated castor oil, alkylallyl formaldehyde condensed polyoxyethylene ether, polyoxyethylene polyoxypropylene block copolymers, polyoxyethylene polyoxypropyl alkyl ether, polyethylene glycol fatty acid ester, alkyl glucoside, N-alkyl gluconamide, polyoxyethylene fatty acid amide, polyoxyethylene alkylamine, polyoxyethylene alkyl ether phosphate, polyoxyethylene alkyl allyl ether phosphate, and the like can be used. Among these surfactants, sorbitan fatty acid ester, polyglycerin fatty acid ester, and sucrose fatty acid ester are particularly preferably used from the viewpoint of the dispersion stability of the monomer. These surfactants may be used singly or in combination of two or more kinds thereof.

The amount of the surfactant used is preferably 0.1 to 30 parts by mass, and more preferably 0.3 to 20 parts by mass based on 100 parts by mass of the first monomer component used for the first stage polymerization.

(Polymer-Based Dispersant)

As the dispersion stabilizer, a polymer-based dispersant may be used in combination with the above-described surfactant. Examples of the polymer-based dispersant include maleic anhydride-modified polyethylene, maleic anhydride-modified polypropylene, maleic anhydride-modified ethylene/propylene copolymers, maleic anhydride-modified EPDM (ethylene/propylene/diene/terpolymer), maleic anhydride-modified polybutadiene, maleic anhydride/ethylene copolymers, maleic anhydride/propylene copolymers, maleic anhydride/ethylene/propylene copolymers, maleic anhydride/butadiene copolymers, polyethylene, polypropylene, ethylene/propylene copolymers, oxidized polyethylene, oxidized polypropylene, oxidized ethylene/propylene copolymers, ethylene/acrylic acid copolymers, ethyl cellulose, and ethyl hydroxyethyl cellulose. Among these polymer-based dispersants, maleic anhydride-modified polyethylene, maleic anhydride-modified polypropylene, maleic anhydride-modified ethylene/propylene copolymers, maleic anhydride/ethylene copolymers, maleic anhydride/propylene copolymers, maleic anhydride/ethylene/propylene copolymers, polyethylene, polypropylene, ethylene/propylene copolymers, oxidized polyethylene, oxidized polypropylene, and oxidized ethylene/propylene copolymers are particularly preferably used from the viewpoint of the dispersion stability of the monomer. These polymer-based dispersants may be used singly or in combination of two or more kinds thereof.

The amount of the polymer-based dispersant used is preferably 0.1 to 30 parts by mass, and more preferably 0.3 to 20 parts by mass based on 100 parts by mass of the first monomer component in the first stage.

[Internal Cross-Linking Agent]

Examples of the internal cross-linking agent include di(meth)acrylic acid esters and tri(meth)acrylic acid esters of polyols such as (poly)ethylene glycol [the prefix "(poly)" means the prefix may be present or absent, the same applies below], (poly)propylene glycol, 1,4-butanediol, trimethylolpropane, and (poly)glycerin; unsaturated polyesters prepared by reacting the above-described polyol with an unsaturated acid such as maleic acid or fumaric acid; bisacrylamides such as N,N-methylenebisacrylamide; di(meth)acrylic acid esters and tri(meth)acrylic acid esters prepared by reacting a polyepoxide with a (meth)acrylic acid; di(meth)acrylic acid carbamyl esters prepared by reacting a polyisocyanate such as tolylene diisocyanate or hexamethylene diisocyanate with hydroxyethyl (meth)acrylate; compounds having two or more polymerizable unsaturated groups, such as allylated starch, allylated cellulose, diallyl phthalate, N,N',N"-triallyl isocyanate, and divinylbenzene; diglycidyl compounds such as (poly)ethylene glycol diglycidyl ether, (poly)propylene glycol diglycidyl ether, and (poly)glycerin diglycidyl ether, polyglycidyl compounds such as triglycidyl compounds; epihalohydrin compounds such as epichlorohydrin, epibromhydrin, and $\alpha$-methylepichlorohydrin; compounds having two or more reactive functional groups, such as isocyanate compounds such as 2,4-tolylenediisocyanate and hexamethylene diisocyanate; and oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal cross-linking agents, polyglycidyl compounds are preferably used, and diglycidyl ether compounds are more preferably used, and (poly)ethylene glycol diglycidyl ether, (poly)propylene glycol diglycidyl ether, (poly)glycerin diglycidyl ether are preferably used. These internal cross-linking agents may be used singly or in combination of two or more kinds thereof.

The lower limit of the amount of the internal cross-linking agent used is preferably 0.00001 mol, more preferably 0.00005 mol, still more preferably 0.0001 mol, and particularly preferably 0.0005 mol based on 1 mol of the first monomer component. The upper limit is preferably 0.01 mol, more preferably 0.005 mol, still more preferably 0.003 mol, and particularly preferably 0.002 mol based on 1 mol of the first monomer component.

[Radical Polymerization Initiator]

In the production of the first polymer particle, the first monomer component is polymerized using a radical polymerization initiator. Examples of the radical polymerization initiator include azo-based compounds and peroxides. The azo-based compound and the peroxide can be used in combination. The radical polymerization initiator may be in the form of powder or an aqueous solution.

(Azo-Based Compound)

Examples of the azo-based compound include azo compounds such as 1-{(1-cyano-1-methylethyl)azo}formamide, 2,2'-azobis[2-(N-phenylamidino)propane]dihydrochloride, 2,2'-azobis{2-[N-(4-chlorophenyl)amidino]propane}dihydrochloride, 2,2'-azobis{2-[N-(4-hydroxyphenyl)amidino]propane}dihydrochloride, 2,2'-azobis[2-(N-benzylamidino)propane]dihydrochloride, 2,2'-azobis[2-(N-allylamidino)propane]dihydrochloride, 2,2'-azobis(2-amidinopropane)dihydrochloride, 2,2'-azobis{2-[N-(2-hydroxyethyl)amidino]propane}dihydrochloride, 2,2'-azobis[2-(5-methyl-2-imidazoline-2-yl)propane]dihydrochloride, 2,2'-azobis[2-(2-imidazoline-2-yl)propane]dihydrochloride, 2,2'-azobis[2-(4,5,6,7-tetrahydro-1H-1,3-diazepin-2-yl)propane]dihydrochloride, 2,2'-azobis[2-(5-hydroxy-3,4,5,6-tetrahydropyrimidin-2-yl)propane]dihydrochloride, 2,2'-azobis{2-[1-(2-hydroxyethyl)-2-imidazoline-2-yl]propane}dihydrochloride, 2,2'-azobis[2-(2-imidazoline-2-yl)propane], 2,2'-azobis{2-methyl-N-[1,1-bis(hydroxymethyl)-2-hydroxyethyl]propionamide], 2,2'-azobis{2-methyl-N-[1,1-bis(hydroxymethyl)ethyl]propionamide}, 2,2'-azobis[2-methyl-N-(2-hydroxyethyl)propionamide], 2,2'-azobis(2-methylpropionamide)dihydrochloride, 4,4'-azobis-4-cyanovaleic acid, 2,2'-azobis[2-(hydroxymethyl)propionitrile], 2,2'-azobis[2-(2-imidazoline-2-yl)propane] disulfate dihydrate, 2,2'-azobis[N-(2-carboxyethyl)-2-methylpropionamidine] tetrahydrate, and 2,2'-azobis[2-methyl-N-(2-hydroxyethyl)propionamide]. Among these azo-based compounds, 2,2'-azobis(2-amidinopropane)dihydrochloride, 2,2'-azobis{2-[1-(2-hydroxyethyl)-2-imidazoline-2-yl]propane}dihydrochloride, and 2,2'-azobis[N-(2-carboxyethyl)-2-methylpropionamidine] tetrahydrate are preferable. These azo-based compounds may be used singly or in combination of two or more kinds thereof.

(Peroxide)

Examples of the peroxide include persulfates such as potassium persulfate, ammonium persulfate, and sodium persulfate; and peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide. Among these peroxides, potassium persulfate, ammonium persulfate, sodium persulfate, and hydrogen peroxide are preferably used, and potassium persulfate, ammonium persulfate, and sodium persulfate are more preferably used. These peroxides may be used singly or in combination of two or more kinds thereof.

The amount of the radical polymerization initiator used is preferably 0.00005 mol or more, and more preferably 0.0001 mol or more based on 1 mol of the first monomer component. Furthermore, the amount of the radical polymerization initiator used is preferably 0.005 mol or less, and more preferably 0.002 mol or less based on 1 mol of the first monomer component.

In the case that the azo-based compound and the peroxide are used in combination, the proportion of the amount of the azo-based compound used to the total amount of the azo-based compound and the peroxide used is preferably 40% by mass or more, more preferably 50% by mass or more, still more preferably 60% by mass or more, and still even more preferably 70% by mass or more. Furthermore, the proportion of the amount of the azo-based compound used to the total amount of the azo-based compound and the peroxide used is preferably 95% by mass or less, more preferably 90% by mass or less, still more preferably 85% by mass or less, and still even more preferably 80% by mass or less. The range of the mass ratio (azo-based compound : peroxide) is preferably 8 : 12 to 19 : 1.

[Another Ingredient]

In the production of the first polymer particle, if desired, an additive may be added to the first monomer component to perform reverse phase suspension polymerization. Examples of the additive include chain transfer agents and thickeners.

(Chain Transfer Agent)

For example, the first monomer component may be polymerized in the presence of a chain transfer agent.

Examples of the chain transfer agent include thiols such as ethanethiol, propanethiol, and dodecanethiol; thiol acids such as thioglycolic acid, thiomalic acid, dimethyldithiocarbamic acid, diethyldithiocarbamic acid, and salts thereof; secondary alcohols such as isopropanol; phosphorous acid, normal salts of phosphorous acid, such as disodium phosphite, dipotassium phosphite, and diammonium phosphite, phosphorous acid compounds such as acidic salts of phosphorous acid, such as sodium hydrogen phosphite, potassium hydrogen phosphite, and ammonium hydrogen phosphite; phosphoric acid, normal salts of phosphoric acid, such as sodium phosphate, potassium phosphate, and ammonium phosphate, phosphoric acid compounds such as acidic salts of phosphoric acid, such as sodium dihydrogen phosphate, potassium dihydrogen phosphate, ammonium dihydrogen phosphate, disodium hydrogen phosphate, dipotassium hydrogen phosphate, and diammonium hydrogen phosphate; hypophosphorous acid, hypophosphorous acid compounds such as hypophosphites such as sodium hypophosphite, potassium hypophosphite, and ammonium hypophosphite; pyrophosphoric acid, tripolyphosphoric acid, polyphosphoric acid, salts thereof; trimethyl phosphate, and nitrilotrimethylene triphosphonic acid. These chain transfer agents may be used singly or in combination of two or more kinds thereof. Furthermore, hydrates of the above-described chain transfer agents may be used as a chain transfer agent.

The amount of the chain transfer agent used is preferably 0.00001 to 0.0005 mol, and more preferably 0.000025 to 0.00012 mol based on 1 mol of the first monomer component.

(Thickener)

Furthermore, a thickener may be added to the aqueous solution containing the first monomer component to perform reverse phase suspension polymerization. By adding the thickener in this manner to adjust the viscosity of the aqueous solution, it is also possible to control the median particle diameter obtained in the reverse phase suspension polymerization.

As the thickener, for example, hydroxyethyl cellulose, hydroxypropyl cellulose, methyl cellulose, carboxymethyl cellulose, polyacrylic acid, polyacrylic acid (partial) neutralized products, polyethylene glycol, polyacrylamide, polyethyleneimine, dextrin, sodium alginate, polyvinyl alcohol, polyvinylpyrrolidone, polyethylene oxide, or the like can be used. If the stirring speed during the polymerization is the same, the higher the viscosity of the aqueous solution of the first monomer component is, the larger the median particle diameter of the particle obtained tends to be.

[Reverse Phase Suspension Polymerization]

When the reverse phase suspension polymerization is performed, for example, an aqueous solution containing the first monomer component is dispersed in a hydrocarbon dispersion medium in the presence of a dispersion stabilizer. At this time, the dispersion stabilizer (such as a surfactant or a polymer-based dispersant) may be added before or after dispersing the aqueous solution of the first monomer component in the hydrocarbon dispersion medium as long as the dispersion stabilizer is added before starting the polymerization reaction.

Among the above-described procedures, a procedure is preferable in which the aqueous solution of the first monomer component is dispersed in the hydrocarbon dispersion medium in which the polymer-based dispersant is dispersed, and then a surfactant is further added to perform polymerization from the viewpoint of easily reducing the amount of the hydrocarbon dispersion medium remaining in the first polymer particle to be obtained.

In the method for producing the first polymer particle, the above-described reverse phase suspension polymerization can be performed in one stage or in multiple stages of two or more stages. From the viewpoint of improving the productivity, the reverse phase suspension polymerization may be performed in 2 to 3 stages.

In the case of reverse phase suspension polymerization in multiple stages of two or more stages, it is required that after reverse phase suspension polymerization is performed in the first stage, the first monomer component is added and mixed into the reaction mixture obtained in the first stage polymerization reaction, and reverse phase suspension polymerization is performed in the second and the subsequent stages in the same manner as in the first stage. In the reverse phase suspension polymerization in each of the second and the subsequent stages, it is preferable that the above-described internal cross-linking agent, azo compound, peroxide, and the like that are added if necessary in addition to the first monomer component be added in the range of the above-described molar ratio of each component to the first monomer component based on the amount of the first monomer component to be added in the reverse phase suspension polymerization in each of the second and the subsequent stages, and reverse phase suspension polymerization be performed. In the production of the first polymer particle, polymerization is preferably performed in the presence of at least one of the azo-based compound or the peroxide even in the second and the subsequent stages.

The reaction temperature of the polymerization reaction of the first monomer component is preferably 20 to 110°C, and more preferably 40 to 90°C from the viewpoints of rapidly advancing the polymerization and shortening the polymerization time to enhance the economical efficiency, and easily removing the polymerization heat to smoothly perform the reaction. The reaction time is preferably 0.1 to 4 hours.

As described above, the first polymer particle can be suitably produced. The median particle diameter of the first polymer particle is required to be appropriately adjusted so that the median particle diameter of the water-absorbing exfoliator obtained after the infiltration of the second polymer is in the above-described range, and for example, the median particle diameter of the first polymer particle is preferably 1 to 450 μm.

<Step of Infiltrating Second Monomer Component>

Next, a second monomer component that is to form a second polymer and includes at least one of a monomer B or a salt of the monomer B is infiltrated into the first polymer particle. The second monomer component may include only the monomer B, may include only a salt of the monomer B, may include only the monomer B and its salt, or may include a monomer, other than the monomer B and its salt, (hereinafter referred to as monomer Y) in addition to the monomer B and/or its salt.

As the monomer B, a monomer satisfying the above-described acid dissociation index is preferable, and a water-soluble ethylenically unsaturated monomer is preferable. Examples of the water-soluble ethylenically unsaturated monomer include (meth)acrylic acid; nonionic monomers such as (meth)acrylamide, N,N-dimethyl(meth)acrylamide, 2-hydroxyethyl (meth)acrylate, N-methylol

(meth)acrylamide, and polyethylene glycol mono(meth)acrylate; unsaturated monomers including an amino group, and quaternary compounds thereof, such as N,N-diethylaminoethyl (meth)acrylate, N,N-diethylaminopropyl (meth)acrylate, and diethylaminopropyl (meth)acrylamide. Among these water-soluble ethylenically unsaturated monomers, (meth)acrylic acid, (meth)acrylamide, N,N-dimethylacrylamide are preferable, and (meth)acrylic acid is more preferable from the viewpoints of industrial availability and the like. Only one of the monomers B may be used, or two or more of the monomers B may be used in combination.

In the case that two or more monomers B are used in combination, it is preferable that one or some monomers B have an acid dissociation index in the above-described range, and it is more preferable that all monomers B have an acid dissociation index in the above-described range.

In the case that two or more monomers B are used in combination, the reference monomer B for determining the difference in acid dissociation index from the monomer A ($\Delta$pKa) is the monomer B having the smallest acid dissociation index of the two or more monomers B.

As for the monomer Y, only one monomer Y may be used, or two or more monomers Y may be mixed and used. For example, in the case that a water-soluble ethylenically unsaturated monomer such as (meth)acrylic acid is used as the monomer B, the above-described monomer A (for example, allyl sulfonic acid and/or methallyl sulfonic acid as an unsaturated sulfonic acid-based monomer) may be used as the monomer Y. In the second monomer component, the proportion of the monomer B and/or its salt (in the case that the monomer B and its salt are included, the proportion of the total amount of the monomer B and its salt) is preferably 70 mol% or more, more preferably 80 mol% or more, and still more preferably 90 mol% or more. The upper limit of the proportion of the monomer B and/or its salt is 100 mol%. If the proportion of the monomer B and/or its salt is 100 mol%, the second monomer component includes no monomer Y.

In the case that the monomer B, like (meth)acrylic acid, has an acid group, the salt of the monomer B can be prepared by neutralizing the acid group with an alkaline neutralizing agent. Examples of the alkaline neutralizing agent include the same neutralizing agents as those used for neutralizing the monomer A described above.

The degree of neutralization of the monomer B with the alkaline neutralizing agent is preferably 10 to 100 mol%, more preferably 30 to 90 mol%, still more preferably 40 to 85 mol%, and still even more preferably 50 to 80 mol% as the degree of neutralization based on all the acid groups of the monomer B.

As a method of infiltrating the second monomer component into the first polymer particle, for example, the first polymer particle and the second monomer component are required to be mixed. At this time, in order to sufficiently infiltrate the second monomer component into the inside of the first polymer particle, the first polymer particle is preferably dried. The first polymer particle can be dried in the same manner as in the step of drying the water-absorbing exfoliator described below.

In order to sufficiently infiltrate the second monomer component into the inside of the first polymer particle, the second monomer component is preferably infiltrated into the first polymer particle in the form of an aqueous solution. More specifically, the first polymer particle is immersed in an aqueous solution of the second monomer component to suitably infiltrate the second monomer component into the first polymer particle. The immersion time is, for example, 0.3 to 48 hours.

The concentration of the second monomer component in the aqueous solution of the second monomer component is preferably in the range of 20% by mass to the saturated concentration. The concentration of the second monomer component is more preferably 55% by mass or less, still more preferably 50% by mass or less, and still even more preferably 45% by mass or less. Furthermore, the concentration of the second monomer component is more preferably 25% by mass or more, still more preferably 28% by mass or more, and still even more preferably 30% by mass or more.

From the viewpoint of suitably polymerizing the second monomer component infiltrated into the first polymer particle, the second monomer component may be infiltrated into the first polymer particle in the form of an aqueous dispersion in which at least one component of an internal cross-linking agent, an azo-based compound, a peroxide, or the like is further dispersed. Examples of the component include the same components as those exemplified in the step of polymerizing the first monomer component described above. The amount of the component used can be the same as those exemplified for the first monomer component described above.

<Step of Polymerizing Second Monomer Component>

Next, the second monomer component infiltrated into the first polymer particle is polymerized to obtain a water-absorbing exfoliator having a structure in which the second polymer is infiltrated into the first polymer particle.

The second monomer component, like the above-described first monomer component, can be polymerized under the condition of reverse phase suspension polymerization. That is, for example, the first polymer particle into which the second monomer component is infiltrated is dispersed in a hydrocarbon dispersion medium in the presence of a dispersion stabilizer. At this time, the dispersion stabilizer (such as a surfactant or a polymer-based dispersant) may be added before or after dispersing the first polymer particle into which the second monomer component is infiltrated in the hydrocarbon dispersion medium as long as the dispersion stabilizer is added before starting the polymerization reaction of the second monomer component. As the hydrocarbon dispersion medium and the dispersion stabilizer, the same ones as those exemplified in the step of polymerizing the first monomer component described above are used, and the amounts of the hydrocarbon dispersion medium and the dispersion stabilizer used can be the same as those exemplified for the first monomer component described above.

From the viewpoint of easily reducing the amount of the hydrocarbon dispersion medium remaining in the water-absorbing exfoliator to be obtained, it is preferable that the first polymer particle into which the second monomer component is infiltrated be dispersed in the hydrocarbon dispersion medium in which the polymer-based dispersant is dispersed, and then a surfactant be further added to perform polymerization.

The reaction temperature of the polymerization reaction of the second monomer component is preferably 20 to 110°C, and more preferably 40 to 90°C from the viewpoints of rapidly advancing the polymerization and shortening the polymerization time to enhance the economical efficiency, and easily removing the polymerization heat to smoothly perform the reaction. The reaction time is preferably 0.5 to 4 hours.

<Step of Post-Crosslinking>

In the method for producing the water-absorbing exfoliator, it is also possible to subject the water-containing gel-like material of the water-absorbing exfoliator that is obtained by the above-described method and has a structure in which the second polymer is infiltrated into the first polymer particle to post-crosslinking with a post-crosslinking agent (post-crosslinking reaction). The post-crosslinking reaction is preferably performed in the presence of a post-crosslinking agent after the polymerization of the second monomer component. Thus, by subjecting the water-containing gel-like material of the water-absorbing exfoliator to post-crosslinking reaction after the polymerization, the cross-linking density in the vicinity of the surface of the water-absorbing exfoliator is increased, and the hardness can be adjusted to a further appropriate range so that the massage effect is excellent.

Examples of the post-crosslinking agent include compounds having two or more reactive functional groups. The examples include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromhydrin, and $\alpha$-methyl epichlorohydrin; isocyanate compounds such as 2,4-tolylene diisocyanate and hexamethylene diisocyanate; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. Among these post-crosslinking agents, polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether are preferable. These post-crosslinking agents may be used singly or in combination of two or more kinds thereof.

The amount of the post-crosslinking agent used is preferably 0.00001 to 0.01 mol, more preferably 0.00005 to 0.005 mol, and still more preferably 0.0001 to 0.002 mol based on 1 mol of the second monomer component used in the polymerization.

In the method of adding the post-crosslinking agent, the post-crosslinking agent may be added as it is or as an aqueous solution, and if necessary, may be added as a solution in which a hydrophilic organic solvent is used as a solvent. Examples of the hydrophilic organic solvent include lower alcohols such as methyl alcohol, ethyl alcohol, n-propyl alcohol, and isopropyl alcohol; ketones such as acetone and methyl ethyl ketone; ethers such as diethyl ether, dioxane, and tetrahydrofuran; amides such as N,N-dimethylformamide; and sulfoxides such as dimethyl sulfoxide. These hydrophilic organic solvents may be used singly, in combination of two or more kinds thereof, or as a mixed solvent with water.

The post-crosslinking agent is required to be added after the polymerization reaction of the second monomer component is almost completed. The post-crosslinking agent is preferably added in the presence of water in the range of 1 to 400 parts by mass, more preferably in the presence of water in the range of 5 to 200 parts by mass, still more preferably in the presence of water in the range of 10 to 100 parts by mass, and still even more preferably in the presence of water in the range of 20 to 70 parts by mass based on 100 parts by mass of the total amount of the first monomer component and the second monomer component. The amount of water means the total amount of water included in the polymerization reaction system and water used if necessary when the post-crosslinking agent is added.

The reaction temperature in the post-crosslinking reaction is preferably 50 to 250°C, more preferably 60 to 180°C, still more preferably 60 to 140°C, and still even more preferably 70 to 120°C. The reaction time of the post-crosslinking reaction is preferably 1 to 300 minutes, and more preferably 5 to 200 minutes.

<Step of Drying>

The method for producing the water-absorbing exfoliator may include a step of drying after the polymerization of the second monomer component. In the step of drying, for example, after the second monomer component is polymerized, energy such as heat is added from the outside to the system to remove the water, the hydrocarbon dispersion medium, and the like from the system (the container in which the reaction is performed) by distillation. When the water-containing gel after the polymerization is dehydrated, the system in which the water-containing gel is dispersed in the hydrocarbon dispersion medium is heated to once distill the water and the hydrocarbon dispersion medium out of the system by azeotropic distillation. At this time, azeotropic distillation can be continuously performed by returning only the distilled hydrocarbon dispersion medium into the system. In this case, the temperature in the system during the drying is maintained at the azeotropic temperature or lower with the hydrocarbon dispersion medium, so that the resin is difficult to deteriorate. The water and the hydrocarbon dispersion medium are subsequently distilled out to obtain a water-absorbing exfoliator.

The step of drying may be performed under normal pressure or reduced pressure. From the viewpoint of enhancing the drying efficiency, the step of drying may be performed under a stream of nitrogen or the like. In the case that the step of drying is performed under normal pressure, the drying temperature is preferably 70 to 250°C, more preferably 80 to 180°C, still more preferably 80 to 140°C, and still even more preferably 90 to 130°C. In the case that the step of drying is performed under reduced pressure, the drying temperature is preferably 40 to 160°C, and more preferably 50 to 120°C.

In the case that the step of post-crosslinking with the post-crosslinking agent is performed, the above-described step of drying is preferably performed after the step of post-crosslinking.

The water-absorbing exfoliator may include an additive depending on the purpose. Examples of such an additive include inorganic powders, surfactants, oxidizing agents, reducing agents, metal chelating agents, radical chain inhibitors, antioxidants, antibacterial agents, and deodorants. Furthermore, there is a possibility that the water-absorbing exfoliator will include various components used in the polymerization of the first polymer particle and the second polymer (for example, the hydrocarbon dispersion medium, the dispersion stabilizer, the internal cross-linking agent, the azo-based compound, the peroxide, the chain transfer agent, and the thickener) and their reactants.

(Cosmetic)

The cosmetic according to the present invention includes the above-described water-absorbing exfoliator according to the present invention.

The water-absorbing exfoliator according to the present invention may be used as a simple substance (single particle) or may be used in a plurality (as aggregate of single particles), and usually, the plurality of the water-absorbing exfoliators are blended with a cosmetic and used. The cosmetic according to the present invention may include an exfoliator other than the water-absorbing exfoliator according to the present invention. The total amount of the exfoliator included in the cosmetic according to the present invention is, for example, 0.1 to 10% by mass, preferably 0.3 to 7.0% by mass, and more preferably 0.5 to 5.0% by mass. It is preferable that 50% by mass or more of the exfoliator included in the cosmetic be the water-absorbing exfoliator according to the present invention, more preferable that 80% by mass or more be the water-absorbing exfoliator according to the present invention, and still more preferable that 100% by mass be the water-absorbing exfoliator according to the present invention.

Examples of the exfoliator, that can be included in the cosmetic, other than the water-absorbing exfoliator according to the present invention include particles of activated carbon, pulverized products of talc, particles of a synthetic resin such as polyethylene, and spherical porous silica.

The cosmetic according to the present invention can include, in addition to the water-absorbing exfoliator, various components included in known cosmetics. The components are not particularly limited, and examples of the components include cleaning components, solvents, water, oily components, fragrances, colorants, surfactants, alcohols, thickeners, gelling agents, ultraviolet absorbers, antioxidants, moisturizers, antiseptics, antibacterial agents, pH adjusters, whitening agents, vitamins, cooling agents, and blood circulation promoters. Only one of the components included in the cosmetic may be used, or two or more of the components may be mixed and used.

The dosage form of the cosmetic according to the present invention is not particularly limited, and a known cosmetic dosage form can be used. Specific examples of the dosage form include liquid forms, cream forms, and gel forms.

The cosmetic according to the present invention can be suitably used as, for example, a face wash, a hair wash, a skin cleaning agent for the other body parts, a massage cosmetic, a skin care cosmetic, and the like.

Examples

Hereinafter, the present invention will be described in detail with reference to Examples and Comparative Examples. However, the present invention is not limited to Examples. The pure-water absorption factor, the compression-breaking stress, and the pKa were measured by the following methods. The exfoliator was evaluated by the following panelist evaluation method.

<Method of Measuring Pure-Water Absorption Factor>

The prepared water-absorbing exfoliator was classified into particles that passed through a JIS standard sieve having a mesh-opening of 250 $\mu$m and remained on a JIS standard sieve having a mesh-opening of 150 $\mu$m. In a 500 mL beaker, 500 g of pure water was weighed and put, and 0.25 $\pm$ 0.0002 g of the water-absorbing exfoliator classified into particles having a size of 150 $\mu$m to 250 $\mu$m was dispersed in the pure water while the mixture was stirred at a stirring speed of 600 rpm with a magnetic stirrer bar (having a size of 8 mmcp $\times$ 30 mm without a ring) so that no lump was generated. The mixture was left for 30 minutes in a state of being stirred to swell the water-absorbing exfoliator sufficiently. Then, the mass Wa (g) of a JIS standard sieve having a mesh-opening of 75 $\mu$m was measured in advance, and using the sieve, the contents of the beaker were filtered, and the sieve was left inclined by about 30 degrees with respect to the horizontal for 30 minutes to remove the excess water. The mass Wb (g) of the sieve containing the water-absorbing gel was measured, and the pure-water absorption factor was determined by the following formula.

$$\text{Pure-water absorption factor (g/g)} = [\text{Wb - Wa}] \text{ (g)/mass of water-absorbing}$$

$$\text{exfoliator (g)}$$

<Method of Measuring Compression-Breaking Stress>

The prepared water-absorbing exfoliator was classified into particles that passed through a JIS standard sieve having a mesh-opening of 250 $\mu$m and remained on a JIS standard sieve having a mesh-opening of 150 $\mu$m. In a 10 mL beaker, 3.0 g of pure water was weighed and put, and 0.10 $\pm$ 0.0002 g of the classified water-absorbing exfoliator was added in the pure water while the mixture was stirred with a magnetic stirrer bar (having a size of 4 mmcp $\times$ 11 mm without a ring). The mixture was left for 2 minutes in a state of being stirred, then the stirring was stopped, and the mixture was left for another 30 minutes to swell the water-absorbing exfoliator to 30 times by mass to obtain a water-absorbing gel. Using a compact compression/tensile tester ("Eztest/CE" manufactured by SHIMADZU CORPORATION), the load required to break one particle of the water-absorbing gel was measured in accordance with the following operations and conditions. One particle of the water-absorbing gel was placed on the center of the measuring table, an indenter was lowered from above the water-absorbing gel at a constant speed (displacement speed) shown in the conditions described below, and while a graph of the load against the moving distance of the indenter was displayed, the variation of the load was recorded. When the indenter comes into contact with the water-absorbing gel, the repulsive force of the water-absorbing gel increases the load. When the water-absorbing gel is broken, the repulsive force is decreased to decrease the load. Based on the fact, the indenter was moved until the increase in the load caused by lowering the indenter stopped and then the load was decreased (Examples 1, 2, and 3 and Comparative Example 2). In the case that the load was not decreased, the indenter was lowered until the load reached the load limit of the load cell in accordance with the conditions (Comparative Example 1). The maximum load immediately before the decrease in the load occurred was measured and regarded as the yield load of the water-absorbing gel. This yield load was determined to be the load required to break the water-absorbing gel. The yield load of each of three particles of the water-absorbing gel was measured using a load cell A or a load cell B (the load cell A was used in Comparative Example 1, and the load cell B was used in Examples 1 to 3 and Comparative Example 2), and the compression-breaking stress (N) was determined from the average of the measured values. The value of the compression-breaking stress is one of the indexes showing the strength of the crosslinked polymer, and the higher the compression-breaking stress is, the more difficult the water-absorbing gel tends to be to break.
The conditions of the load cell used in measuring the load are as follows.

Upper limit and lower limit of quantification of load cell A: 20 (N) and 0.4 (N)
Upper limit and lower limit of quantification of load cell B: 2 (N) and 0.04 (N)
Displacement speed: 0.5 (mm/min)
Load limit (upper limit) of load cell A set as measurement condition: 15.0 (N)
Load limit (upper limit) of load cell B set as measurement condition: 1.5 (N)

Indenter: 15 (mmcp)

<Method of Measuring pKa>

To 20.4 g of the monomer whose pKa was to be measured, 29.6 g of ion-exchanged water was weighed and added in a 100 mL glass beaker, and the mixture was mixed for 5 minutes while stirred with a magnetic stirrer bar (having a size of 8 mmcp $\times$ 30 mm without a ring) to prepare 40.8% by mass of an aqueous solution. In a 100 mL glass beaker, 2.4 g of the aqueous solution and 50.0 g of physiological saline were weighed and put, and the mixture was stirred with a magnetic stirrer bar (having a size of 8 mmcp $\times$ 30 mm without a ring) to prepare an aqueous solution for measurement. The aqueous solution for measurement was adjusted to 17°C until immediately before the measurement. The acid dissociation index was measured using an automatic titrator (COM-1600) manufactured by HIRANUMA SANGYO Co., Ltd. While the aqueous solution for measurement was stirred, 0.025 mL of 0.1 M sodium hydroxide was dropped every 10 seconds, and the pH of the aqueous solution for measurement was measured in each dropping. The acid dissociation index pKa of the monomer was determined using the Henderson-Hasselbalch equation from the concentration of the aqueous solution for measurement, the amount of the dropped sodium hydroxide, and the measured pH.

<Panelist Evaluation Method>

A cosmetic was prepared using the exfoliator. Specifically, the exfoliator was classified into particles that passed through a JIS standard sieve having a mesh-opening of 250 $\mu$m and remained on a JIS standard sieve having a mesh-opening of 150 $\mu$m. Next, components 1 and 2 were first mixed so as to have the following composition, the mixture was heated to 70°C and stirred to obtain a uniform mixed liquid. Next, the following components 3 to 6 were added to the obtained mixed liquid, and the mixture was stirred to prepare an emulsion cosmetic (cream).

(Composition of Cosmetic)

| | | |
|---|---|---|
| Component 1. | Polysorbate: 60 | 3 g |
| Component 2. | Purified water: | 66 g |
| Component 3. | Mineral oil: | 10 g |
| Component 4. | Isopropyl myristate: | 10 g |
| Component 5. | Caprylic/capric triglyceride: | 10 g |
| Component 6. | Exfoliator: | 1 g |

Next, 10 panelists evaluated the massage effect and the skin irritation due to the hardness when the obtained cosmetic was applied to the hand and the hand was massaged. The evaluation criteria are as follows, and the average of the evaluation points of 10 panelists is shown in Table 1.

(Evaluation Criteria for Massage Effect)

1 Exfoliator is not tangible and massage effect is low
2 Exfoliator is slightly tangible and massage effect is slightly low
3 Exfoliator is tangible and massage effect is normal
4 Exfoliator is suitably tangible and massage effect is slightly high
5 Exfoliator is particularly suitably tangible and massage effect is high

(Evaluation Criteria for Skin Irritation due to Hardness)

1 Irritation due to hardness of exfoliator is too strong
2 Irritation due to hardness of exfoliator is strong
3 Irritation due to hardness of exfoliator is slightly strong
4 Irritation due to hardness of exfoliator is slightly weak

5 Irritation due to hardness of exfoliator is weak

(Example 1)

A 2L round-bottom cylindrical separable flask having an inner diameter of 110 mm equipped with a reflux condenser, a nitrogen gas introduction tube, and, as a stirrer, a stirring blade having two stages each having four inclined paddle blades having a blade diameter of 50 mm was prepared. In the flask, 293 g of n-heptane was put as a hydrocarbon dispersant, 0.74 g of a maleic anhydride-modified ethylene/propylene copolymer (manufactured by Mitsui Chemicals, Inc., Hi-WAX 1105A) was added as a polymer dispersant and dissolved by heating while the mixture was stirred, and then the mixture was cooled to 58°C.

In a 500 mL Erlenmeyer flask, 82.69 g (0.399 mol, pKa: 1.4) of 2-acrylamidomethylpropanesulfonic acid whose acid dissociation index (pKa) was previously measured by the above-described method was put as a monomer A, and 68.59 g of ion-exchanged water was added to prepare an aqueous solution. While the aqueous solution was cooled from the outside, 51.60 g of a 30% by mass sodium hydroxide aqueous solution was dropped to neutralize 97 mol% of the monomer A, then, 0.08 g of hydroxylethyl cellulose (manufactured by Sumitomo Seika Chemicals Company, Limited, HEC AW-15F) as a thickener, 2.19 g of a 5% by mass 2,2'azobis(2-aziminopropane) dihydrochloride aqueous solution as an azo-based compound, 3.20 g of a 3% by mass N,N'-methylenebisacrylamide aqueous solution as an internal cross-linking agent, and 32.40 g of ion-exchanged water were added, and the solutes were dissolved to prepare an aqueous solution containing, as the first monomer component, the monomer A and its sodium salt.

Then, the aqueous solution, prepared as described above, containing the first monomer component was added to the separable flask, and the mixture was stirred at a stirring speed of 300 rpm for 10 minutes while nitrogen was passed through the system at a rate of 0.2 L/min. Separately, in 6.62 g of n-heptane, 0.74 g of sucrose stearate having an HLB of 3 (manufactured by Mitsubishi-kagaku Foods Corporation, RYOTO Sugar Ester S-370) as a surfactant was dissolved by heating to prepare 7.36 g of a surfactant solution. The surfactant solution was further added to the aqueous solution containing the first monomer component, the atmosphere inside the system was sufficiently replaced with nitrogen while the mixed solution was stirred at a stirring speed of 550 rpm for 20 minutes, then the separable flask was immersed in a water bath at 70°C to raise the temperature, and polymerization was performed for 26 minutes.

After the first stage polymerization, 110 g of n-heptane was added to the system, the stirring speed was changed to 1,000 rpm, then the reaction solution was heated in an oil bath at 125°C, and 121.15 g of water was removed out of the system by azeotropic distillation of n-propane and water while n-heptane was refluxed. Then, n-heptane was evaporated, and the resulting product was dried to obtain a dried product of the crosslinked polymer (first polymer particle).

JIS standard sieves having a mesh-opening of 425 μm, 300 μm, 250 μm, 180 μm, 106 μm, 75 μm, and 45 μm, and a saucer were combined in this order from the top. In the combined uppermost sieve, 10 g of the first polymer particles were put and rubbed on sieves by hand in order from the sieve having the largest mesh-opening to disentangle the partially observed agglomeration of the first polymer particles.

Using ROBOT SIFTER RPS 205 manufactured by Seishin Enterprise Co., Ltd., under the conditions of a sound wave intensity of 40 W/m$^2$, a frequency of 80 Hz, a pulse interval of 1 second, and a classification time of 2 minutes, JIS standard sieves for ROBOT SIFTER having a mesh-opening of 425 μm, 300 μm, 250 μm, 180 μm, 106 μm, 75 μm, and 45 μm were combined from the top, and the disentangled first polymer particles were put in a filling container of the apparatus and classified. After the classification, the mass of the first polymer particle remaining on each sieve was calculated as a mass percentage based on the total amount, and the particle size distribution was obtained. Based on the particle size distribution, the mass percentages on the sieves were accumulated in descending order by the particle diameter to plot the relationship between the mesh-opening of the sieve and the accumulated value of the mass percentage of the water-absorbing resin remaining on the sieve on a logarithmic probability paper. The plotted points on the probability paper were connected with a straight line to obtain a particle diameter corresponding to 50% cumulative percentage by mass as the median particle diameter. As a result, the median particle diameter of the obtained first polymer particle was 83 μm.

Acrylic acid (pKa: 4.1) whose acid dissociation index (pKa) was previously measured by the above-described method was prepared as the monomer B and dissolved in ion-exchanged water to prepare 384.6 g of an 80% by mass acrylic acid aqueous solution (containing 4.27 mol of acrylic acid). The aqueous solution was put in a 2L round-mouth wide plastic container, and while the aqueous solution was cooled from the outside, 416.7 g (3.20 mol) of a 30% by mass sodium hydroxide aqueous solution was dropped to neutralize 75 mol% of the acrylic acid aqueous solution. In this container, 6.42 g of a 5% by mass 2,2'azobis(2-aziminopropane) dihydrochloride aqueous solution as an azo-based compound, 6.17 g of a 1% by mass N,N'-methylenebisacrylamide aqueous solution as an internal cross-linking agent, and 164.78 g of ion-exchanged water were added, and the solutes were dissolved to prepare an aqueous solution containing, as the second monomer component, the monomer B and its sodium salt. In the aqueous solution containing the second monomer component, 15 g of the first polymer particle was immersed and sufficiently swollen in a refrigerator for 14 hours. The swollen first polymer particle and the excess aqueous solution containing the second monomer component were separated using a sieve having a mesh-opening of 38 μm. The swollen first polymer particle absorbed 363.69 g of the aqueous solution containing the second monomer component.

A 2L round-bottom cylindrical separable flask having an inner diameter of 110 mm equipped with a reflux condenser, a nitrogen gas introduction tube, and, as a stirrer, a stirring blade having two stages each having four inclined paddle blades having a blade diameter of 50 mm was prepared. In the flask, 293 g of n-heptane was put as a hydrocarbon dispersant, 0.74 g of a maleic anhydride-modified ethylene/propylene copolymer (manufactured by Mitsui Chemicals, Inc., Hi-WAX 1105A) was added as a polymer dispersant and dissolved by heating while the mixture was stirred at a stirring speed of 300 rpm, and then the mixture was cooled to 60°C.

After the cooling to 60°C, 7.36 g of a surfactant solution, that was prepared by dissolving 0.74 g of sucrose stearate having an HLB of 3 (manufactured by Mitsubishi-kagaku Foods Corporation, RYOTO Sugar Ester S-370) as a surfactant in 6.62 g of n-heptane by heating, was further added, and the mixture was stirred for 10 minutes.

After the stirring, 362.02 g of the swollen first polymer particle was added to the system, and the atmosphere inside the system was sufficiently replaced with nitrogen at a rate of 0.2 L/min while the mixture was stirred at a stirring speed of 1,000 rpm for 30 minutes. Then, the flask was immersed in a water bath at 80°C to raise the temperature, the second stage polymerization was performed for 66 minutes, and the second monomer component infiltrated into the first polymer particle was polymerized to obtain a water-absorbing exfoliator having a structure in which the second polymer was infiltrated into the first polymer particle.

After the second stage polymerization, the reaction solution was heated in an oil bath at 125°C, 171.8 g of water was removed out of the system by azeotropic distillation of n-heptane and water while n-heptane was refluxed, then n-heptane was distilled, and the resulting product was dried to obtain a dried product of the water-absorbing exfoliator. The dried product was passed through a sieve having a mesh-opening of 850 μm to obtain 144.22 g of a dried water-absorbing exfoliator.

JIS standard sieves having a mesh-opening of 425 μm, 250 μm, 180 μm, 106 μm, 75 μm, and 45 μm, and a saucer were combined in this order from the top. In the combined uppermost sieve, 50 g of the water-absorbing exfoliator was put and shaken for classification for 10 minutes using a ro-tap shaker. After the classification, the mass of the water-absorbing exfoliator remaining on each sieve was calculated as a mass percentage based on the total amount, and the particle size distribution was obtained. Based on the particle size distribution, the mass percentages on the sieves were accumulated in descending order by the particle diameter to plot the relationship between the mesh-opening of the sieve and the accumulated value of the mass percentage of the water-absorbing exfoliator remaining on the sieve on a logarithmic probability paper. The plotted points on the probability paper were connected with a straight line to obtain a particle diameter corresponding to 50% cumulative percentage by mass as the median particle diameter. As a result, the median particle diameter of the water-absorbing exfoliator at this time was 174 μm.

The obtained water-absorbing exfoliator was classified into particles that passed through a JIS standard sieve having a mesh-opening of 500 μm and remained on a JIS standard sieve having a mesh-opening of 106 μm.

In a 2L round-bottom cylindrical separable flask having an inner diameter of 110 mm equipped with a fluorine resin anchor-shaped stirring blade having a blade diameter of 90 mm, 30 g of the classified water-absorbing exfoliator was put, a solution, that was prepared by mixing 0.02 g of ethylene glycol diglycidyl ether and 0.3 g of propylene glycol that are post-crosslinking agents, 1.0 g of ion-exchanged water, and 0.3 g of isopropanol, was dropped while the mixture was stirred at 500 rpm, and the mixture was stirred for another 1 minute. The polymer was spread on a fluorine resin-coated tray having a bottom surface of 26 × 20 cm and a depth of about 5 cm, and the tray was allowed to stand in a hot air dryer (manufactured by ADVANTEC CO., LTD., FV-320) set at 180°C for 40 minutes. Thus, the water-absorbing exfoliator was crosslinked with the post-crosslinking agent and then passed through a sieve having a mesh-opening of 500 μm to obtain 29 g of the water-absorbing exfoliator having a structure in which the second polymer was infiltrated into the first polymer particle.

Then, the pure-water absorption factor and the compression-breaking stress of the prepared water-absorbing exfoliator were measured by the above-described method, and the water-absorbing exfoliator was evaluated by panelists. The results are shown in Table 1.

(Example 2)

A water-absorbing exfoliator was prepared in the same manner as in Example 1, except that the addition amount of the 3% by mass N,N'-methylenebisacrylamide aqueous solution that was the internal cross-linking agent of the first polymer particle was changed to 3.38 g in Example 1.

Then, the pure-water absorption factor and the compression-breaking stress of the prepared water-absorbing exfoliator were measured by the above-described method, and the water-absorbing exfoliator was evaluated by panelists. The results are shown in Table 1.

(Example 3)

A water-absorbing exfoliator was prepared in the same manner as in Example 1, except that the addition amount of the 3% by mass N,N'-methylenebisacrylamide that was the internal cross-linking agent of the first polymer particle was changed to 3.60 g in Example 1.

Then, the pure-water absorption factor and the compression-breaking stress of the prepared water-absorbing exfoliator were measured by the above-described method, and the water-absorbing exfoliator was evaluated by panelists. The results are shown in Table 1.

(Comparative Example 1)

A low-density polyethylene fine particle manufactured by Sumitomo Seika Chemicals Company, Limited (flow beads CL8007, low-density polyethylene spherical particle, particle diameter: 0.6 mm) was prepared and used as the exfoliator in Comparative Example 1.

Then, the pure-water absorption factor and the compression-breaking stress of the prepared exfoliator were measured by the above-described method, and the exfoliator was evaluated by panelists. When the exfoliator was evaluated by panelists, a cosmetic was prepared without classification of the low-density polyethylene fine particles because the particle diameter of the low-density polyethylene fine particle was not increased owing to water absorption. The results are shown in Table 1.

(Comparative Example 2)

A water-absorbing resin particle manufactured by Sumitomo Seika Chemicals Company, Limited (AQUA KEEP SA50II, acrylic acid polymer partial sodium salt crosslinked product, particle diameter: 256 $\mu$m) was prepared and used as the water-absorbing exfoliator in Comparative Example 1.

Then, the pure-water absorption factor and the compression-breaking stress of the prepared water-absorbing exfoliator were measured by the above-described method, and the water-absorbing exfoliator was evaluated by panelists. The results are shown in Table 1.

[Table 1]

| | Pure-water absorption factor (g/g) | Compression-breaking stress (N) | Massage effect | Skin irritation due to hardness |
|---|---|---|---|---|
| Example 1 | 140 | 0.24 | 4 | 5 |
| Example 2 | 114 | 0.38 | 5 | 5 |
| Example 3 | 103 | 0.51 | 5 | 4 |
| Comparative Example 1 | No water absorption | 15 N or more (upper limit or more) | 4 | 1 |
| Comparative Example 2 | 739 | 0.09 | 2 | 5 |

As is clear from the results shown in Table 1, it can be found that the water-absorbing exfoliators in Examples 1 to 3 exhibit suitable hardness by absorbing water, provide little skin irritation, and have a favorable skin massage effect.

**Claims**

1. A water-absorbing exfoliator **characterized by**:

   a pure-water absorption factor of 30 times or more; and
   a compression-breaking stress of 0.14 to 1.40 N in a state that the water-absorbing exfoliator is swollen to have a mass 30 times of an initial mass of the water-absorbing exfoliator by absorbing water.

2. The water-absorbing exfoliator according to claim 1, having a structure in which a second polymer is infiltrated into a first polymer particle.

3. The water-absorbing exfoliator according to claim 1 or 2, wherein
the first polymer particle includes a polymer of a first monomer component including at least one of a monomer A or a salt of the monomer A,
the second polymer includes a polymer of a second monomer component including at least one of a monomer B or a salt of the monomer B, and
the monomer A has an acid dissociation index smaller than an acid dissociation index of the monomer B.

4. The water-absorbing exfoliator according to claim 3, wherein a difference between the acid dissociation index of the monomer B and the acid dissociation index of the monomer A (ΔpKa) is 1.5 or more.

5. The water-absorbing exfoliator according to claim 4, wherein
the monomer A is an unsaturated sulfonic acid-based monomer, and
the monomer B is a water-soluble ethylenically unsaturated monomer.

6. The water-absorbing exfoliator according to any one of claims 1 to 5, having a granular shape, a substantially spherical shape, or a shape in which substantially spherical particles are aggregated.

7. A method for producing a water-absorbing exfoliator, the method comprising the steps of:

preparing a first polymer particle;
infiltrating a second monomer component that is to form a second polymer and includes at least one of a monomer B or a salt of the monomer B into the first polymer particle; and
polymerizing the second monomer component infiltrated into the first polymer particle to form a structure in which the second polymer is infiltrated into the first polymer particle.

8. A cosmetic comprising the water-absorbing exfoliator according to any one of claims 1 to 7.

## INTERNATIONAL SEARCH REPORT

| | International application No. |
|---|---|
| | PCT/JP2019/013115 |

**A. CLASSIFICATION OF SUBJECT MATTER**
Int.Cl. A61K8/81(2006.01)i, A61Q19/10(2006.01)i

According to International Patent Classification (IPC) or to both national classification and IPC

**B. FIELDS SEARCHED**

Minimum documentation searched (classification system followed by classification symbols)
Int.Cl. A61K8/81, A61Q19/10, C08F2/32

| Documentation searched other than minimum documentation to the extent that such documents are included in the fields searched | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2008-133197 A (SANYO CHEMICAL INDUSTRIES, LTD.) 12 June 2008, claims 1-5, paragraphs [0007], [0014]-[0015] (Family: none) | 1-8 |
| A | JP 2013-199444 A (SUMITOMO SEIKA CHEMICALS CO., LTD.) 03 October 2013, claim 1, paragraphs [0007], [0014], [0024], [0042] (Family: none) | 1-8 |

☒ Further documents are listed in the continuation of Box C.　　☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 21 June 2019 (21.06.2019) | 02 July 2019 (02.07.2019) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

## INTERNATIONAL SEARCH REPORT

International application No.

PCT/JP2019/013115

C (Continuation). DOCUMENTS CONSIDERED TO BE RELEVANT

| Category* | Citation of document, with indication, where appropriate, of the relevant passages | Relevant to claim No. |
|---|---|---|
| A | WO 2007/116554 A1 (ASAHI KASEI CHEMICALS CORP.) 18 October 2007, claims, paragraphs [0026]-[0028], [0040]-[0041], [0065], [0092] & US 2009/0169891 A1, paragraphs [0043]-[0046], [0065]-[0066], [0115], [0165], claims 1-14 & EP 2006306 A1 & KR 10-2008-0094929 A & CN 101410419 A | 1-8 |
| A | JP 2006-089525 A (SUMITOMO SEIKA CHEMICALS CO., LTD.) 06 April 2006, claim 1, paragraphs [0014], [0045] (Family: none) | 1-8 |
| A | JP 05-017509 A (MITSUBISHI YUKA KABUSHIKI KAISHA) 26 January 1993, claims 1-2, paragraph [0017] & US 5548047 A, column 4, lines 13-21, claims 1-8 & EP 522570 A1 & KR 10-0204464 B1 | 1-8 |
| A | JP 01-126314 A (NIPPON SHOKUBAI KAGAKU KOGYO CO., LTD.) 18 May 1989, claims (Family: none) | 1-8 |

Form PCT/ISA/210 (continuation of second sheet) (January 2015)

## INTERNATIONAL SEARCH REPORT

| International application No. |
|---|
| PCT/JP2019/013115 |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
 See extra sheet

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.

PCT/JP2019/013115

<Continuation of Box No. III>

Document 1: JP 2008-133197 A (SANYO CHEMICAL INDUSTRIES, LTD.) 12 June 2008, claims 1-5, paragraphs [0007], [0014]-[0015] (Family: none)

The claims are classified into the two inventions below.

(Invention 1) Claims 1-6 and claim 8 referring to claims 1-6
Claims 1-6 and claim 8 referring to claims 1-6 have the special technical feature of a "water absorptive scrubbing agent of which the pure water absorption ratio is at least 30 times, and the compressive rupture stress in the state of being swollen by 30 mass times by absorbing water is 0.14-1.40 N," and are thus classified as invention 1.

(Invention 2) Claim 7 and claim 8 referring to claim 7
Claim 7 and claim 8 referring to claim 7 share the technical feature of a "water absorptive scrubbing agent" with claim 1 classified as invention 1. However, said technical feature does not make a contribution over the prior art in light of the disclosure (particularly, see [claim 1]) of document 1, and thus cannot be said to be a special technical feature. In addition, there do not exist other identical or corresponding special technical features between claim 7 and claim 8 referring to claim 7, and claim 1.
In addition, claim 7 and claim 8 referring to claim 7 are not dependent on claim 1. In addition, claim 7 and claim 8 referring to claim 7 are not substantially identical or equivalent to any of the claims classified as invention 1.
Thus, claim 7 and claim 8 referring to claim 7 cannot be classified as invention 1.

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6179892 A

- JP 2011225548 A